# EUROPEAN PATENT SPECIFICATION

(11) **EP 2 083 920 B1**
(45) Date of publication and mention of the grant of the patent: **05.10.2011**
(21) Application number: 07824448.0
(22) Date of filing: 05.11.2007
(51) Int. Cl.: A61P 11/02, A61P 11/04, A61P 11/14, A61K 31/164, A61P 17/04, A61P 29/00, C07C 233/57

(54) **N-ARYL-HYDROXYALKYLIDENE-CARBOXAMIDE COMPOUNDS AND THEIR USE**
N-ARYL-HYDROXYALKYLIDEN-CARBONSÄUREAMID-VERBINDUNGEN UND IHRE VERWENDUNG
COMPOSÉS DE N-ARYL-HYDROXYALKYLIDÈNE-CARBOXAMIDE ET UTILISATION DE CES DERNIERS

(30) Priority: 08.11.2006 US 857897 P; 29.12.2006 US 648274
(43) Date of publication of application: 05.08.2009
(73) Proprietor: Wei, Edward Tak, Berkeley, CA 94708 (US)
(72) Inventor: Wei, Edward Tak, Berkeley, CA 94708 (US)
(74) Representative: Florence, Julia Anne
(86) International application number: PCT/GB2007/004212
(87) International publication number: WO 2008/056118

(56) References cited:
- WO-A2-2005/020897
- US-A1- 2005 187 211
- WATSON H R ET AL: "NEW COMPOUNDS WITH THE MENTHOL COOLING EFFECT" JOURNAL OF THE SOCIETY COSMETIC CHEMISTS, SOCIETY OF COSMETIC CHEMISTS,, US, vol. 29, no. 4, 1978, pages 185-200, XP008087152 ISSN: 0037-9832

## Description

### TECHNICAL FIELD

This disclosure generally relates generally to chemicals that affect sensory processes. More particularly, this present invention relates to the peripheral sensory compound 2-Isopropyl-5-methyl-cyclohexanecarboxylic acid [2'-hydroxy-2'-(3"-hydroxy-phenyl)-ethyl]-N methyl-amide that is useful in sensory refreshment, inhibit the perception of itch and pain, and alleviation of skin irritation, itch, and pain. This compound has surprising pharmacokinetic properties that allow a prolonged duration of pharmacological actions and may be administered topically or systemically.

### BACKGROUND

A number of patents and publications are cited herein in order to more fully describe and disclose the invention and the state of the art to which the invention pertains.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

This disclosure includes information that may be useful in understanding the present invention.

About two to three decades ago, a group of scientists discovered novel compounds that have a physiological cooling action on the skin. These were described in U.S. Patent 4,193,936 (Watson et al., March 18, 1980), U.S. Patent 4,248,859 (Rowsell et al., February 3, 1981), U.S. Patent 4,318,900 (Rowsell, March 9, 1982), and in Watson et al., 1978, "New compounds with the menthol cooling effect", J. Soc. Cosmet. Chem., Vol. 29. pp. 185-200.

There are currently two major classes of drugs that act peripherally to reduce perception of nociceptive signals in the central nervous system; nociceptive signals being stimuli that cause irritation, itch and pain. One class is the local anesthetics, such as procaine and lidocaine, which inhibit peripheral nerve conduction of nociceptive signals towards the central nervous system. Another class is agents like aspirin and ibuprofen, that inhibit the synthesis of certain prostaglandins. These prostaglandins, when released by tissues during injury or inflammation, lower the threshold of firing of sensory nerve fibers that respond to noxious stimuli. By "peripheral", it is meant that the target of the drug action is located outside the central nervous system, that is, outside of the brain and spinal cord. By "antinociceptive", it is meant that the drug suppresses the psychical and physiological perception of noxious stimuli.

The sensory fibers that code for thermosensation, irritation, itch and pain are small-diameter sensory fibers called Aδ and unmyelinated C fibers. They are also sometimes called polymodal. When tissues are irritated, injured or inflamed, the C fibers are especially activated. When the compounds of this invention are used, cooling and cold sensations are activated, presumably by Aδ afferents. These sensations have the net effect of reducing perception of C fiber activated nociceptive signals.

U.S. Patent 6,919,348 (Wei et al., July 19, 2005) describes 1,2,3,6-tetrahydropyrimidine-2-one compounds useful for itch.

Other molecules investigated by Wei are described in: US 2005/0059639, published March 17, 2005, Ophthalmic Compositions and Methods for Treating Eye Discomfort and Pain; US 2005/0159394, published July 21, 2005, Aryl-Substituted Derivatives of Cycloalkyl and Branched Chain Alkyl Carboxamides and Carboxylic Acids Useful as Antinociceptive Drugs For Peripheral Targets; US 2005/0187211, published Aug. 25, 2005, *N*-Aryls-Carboxamide Compositions and Methods; and WO 2006/103401, N-Alkylcarbonyl-Amino Acid Ester and *N*-Alkylcarbonyl-Amino Lactone Compounds and Their Use, published Oct. 5, 2006.

WO 2005/020897 A2 describes a large number of compounds which apparently act as Trp-p8 modulators, and which apparently are useful in methods for stimulating apoptosis and/or necrosis, and methods of treating cancer and related disorders. The two compounds below are described on page 58 therein as examples of Trp-p8 agonists:

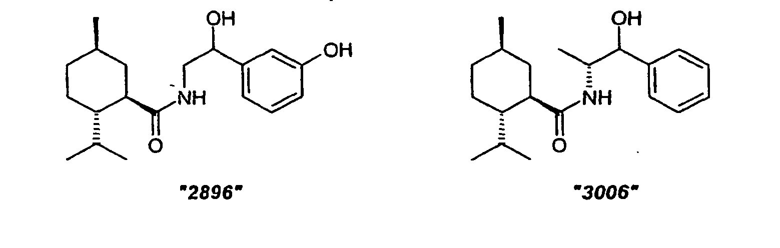

### BRIEF DESCRIPTION OF THE DRAWINGS

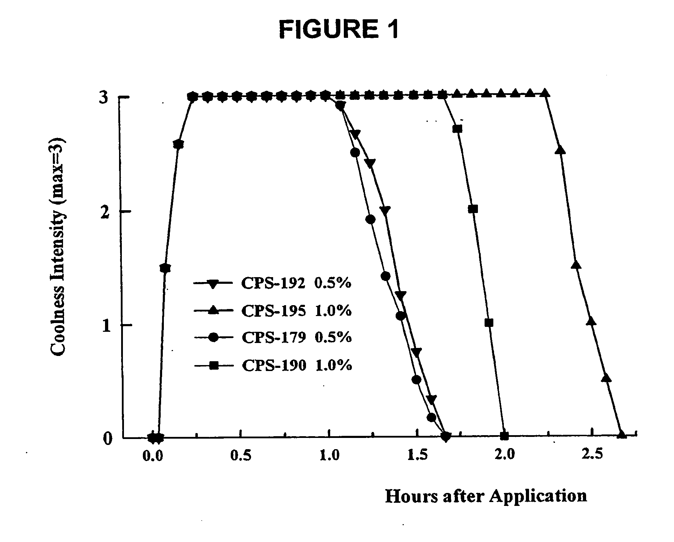

Figure 1 shows a graph of coolness intensity as a function of time after application, for four compounds, CPS-192 (0.5%), CPS-195 (1.0%), CPS-179 (0.5%), and CPS-190 (1.0%), as described in Example 2.

### SUMMARY OF THE INVENTION

One aspect of the present invention pertains to a compound for use in a method of treating sensory discomfort; such as itching; wherein said compound is the compound of the Formula as defined below.

One aspect of the present invention pertains to use of a compound in the manufacture of a medicament for the treatment of sensory discomfort; such as itching; wherein said compound is the compound of the Formula as defined below.

The compound of the invention is:

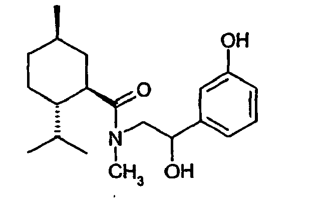

Other aspects of the present disclosure will be understood by reading the following detailed description and the accompanying claims.

### DETAILED DESCRIPTION OF THE INVENTION

Without being bound by theory, the inventor believes that the compound in compositions of the present disclosure act on sensory processes in peripheral neurons to suppress perception of skin irritation, itch, and pain. In normal skin, these compounds generate cooling, refreshing sensations.

Also disclosed are compounds of Formula 1:
R-CO-N(R")-R'-Y Formula 1
   wherein:
   R is (1 R,2S,5R)-2-isopropyl-5-methyl-cyclohexyl;
   R" is hydrogen, methyl, ethyl, n-propyl, or isopropyl;
   R' is a divalent C₂ to C₄ hydroxyalkylidenyl radical;
   Y is a substituted-aryl or -heterocyclyl;
   wherein the aryl or heterocyclyl is phenyl, 1-naphthyl, indenyl, azulenyl, heptalenyl, indacenyl, pyridinyl, dihydropyridinyl, pyridazinyl, piperazinyl, pyrmidinyl, pyrazinyl, indolyl, purinyl, indolizinyl, quinolinyl, isoquinolinyl, quinazolinyl, carbazolyl, pyrrolyl, thiazolyl, isothiazolyl, imidazolyl, benzothiophenyl, or phenathridinyl; and
   wherein one to five of the substituent(s) on the aryl or heterocyclyl are one or more of halogen, C₁ to C₈ alkyl, alkenyl, hydroxyl, C₁ to C₈ hydroxyalkyl, C₁ to C₈ alkoxy, C₁ to C₁₀ hydroxyalkyl or polyhydroxyalkyl, C₂ to C₁₀ alkylcarbonyloxy, C₂ to C₁₀ carboxyalkyl or alkylcarboxyalkyl, C₃ to C₁₀ alkylcarbonyloxyalkyl, C₂ to C₈ acyl, amino, C₁ to C₈ alkylamino, C₂ to C₁₀ acylamino, sulfonamido or C₁ to C₈ alkylsufonylamino, N-arylsulfonamido or N-heterocyclylsutfonamido where the aryl or heterocyclyl is phenyl, benzyl, oxazoyl, thiazoyl, pyrimidinyl, pyridazinyl, or 1,2,4-triazinyl, and where the aryl or heterocyclyl moiety is optionally substituted with a) up to three C₁ to C₃ alkyl groups, b) up to three C₁ to C₃ alkoxy groups, c) C₁ to C₈ aminoalkyl or diaminoalkyl, d) C₂ to C₁₀ alkylaminoalkyl, e) C₂ to C₁₀ acylaminoalkyl, f) carboxy, or g) C₂ to C₁₀ alkylcarboxy.

The term "alkylidene" means a straight or branched saturated, aliphatic, divalent radical having the number of carbon atoms indicated.

The carboxamide group is preferably in an equatorial position relative to the plane of the cycloalkyl ring.

The aryl or heterocyclyl ring permits multiple radical insertions, which increases the versatility of the ligand for its receptor.

For many applications, the form of a pharmaceutically acceptable salt of the Formula 1 compound is useful, such as wherein the salt is an alkali or alkaline earth metal salt, an alkyl-substituted ammonium salt, or a quaternary ammonium salt.

A novel feature of the structures described here is that R' of Formula 1 is C₂ to C₄ hydroxyalkylidene. By "hydroxyalkylidene", it is meant a divalent hydroxyl-substituted, straight or branched saturated hydrocarbon chain with 2 to 4 carbon atoms and of molecular formula -CₙH₂ₙO-, where n is 2, 3, or 4. The consequence of this feature is a more prolonged duration of topical action.

Compositions described by Watson et al., vide supra, when applied directly to the skin in a single dose generally act for less than 1 hour. By contrast, the chemicals described here, when applied once topically, have actions that can last for three to six hours or more.

Some examples of compounds of Formula 1 are compounds wherein:
R' is -CH₂-CH(OH)-; R" is -H; Y is phenyl; and substituents on Y are 4"-CH₂OH (CPS-177, reference example);
R' is -CH₂-CH(OH)-; R" is -CH₃; Y is phenyl; and substituents on Y are 3",4"-OH (CPS -190, reference example);
R' is -CH₂-CH(CH₂OH)-; R" is -H; Y is phenyl; and substituents on Y are 3"-CH₂OH and 4"-C(=O)-CH₃(CPS-178, reference example);
R' is -CH₂-CH(OH)-; R" is -CH₃; Y is phenyl; and substituents on Y are 3"-CH₂-CH₂OH and 4"-C(=O)-CH₃(CPS-191, reference example);
R' is -CH₂CH(CH₂OH)-; R" is -H; Y is phenyl; and substituents on Y are 4"-C(=O)-CH₃ (CPS-179, reference example);
R' is -CH₂-CH(OH)-; R" is -H; Y is phenyl; and substituents on Y are 3"-OH (CPS-192, reference example);
R' is -CH₂-CH(OH)-; R" is -CH₃; Y is phenyl; and substituents on Y are 3"-OCH₃ and 4"-C(=O)-CH₃ (CPS-193, reference example);
R' is -CH₂-CH(OH)-; R" is -CH₃; Y is phenyl; and substituents on Y are 3"-OH and 4"-OCH₃(CPS-194, reference example) or
R' is -CH₂CH(OH)-; R" is -CH₃; Y is phenyl; and substituents on Y are 3"-OH (CPS-195, compounds of the invention).

Some preferred examples of compounds of Formula 1 are compounds wherein:
R' is -CH₂-CH(OH)-; R" is -CH₃; Y is phenyl; and substituents on Y are 3",4"-OH (CPS-190, reference example);
R' is -CH₂-CH(CH₂OH)-; R" is -H; Y is phenyl; and substituents on Y are 4"-C(=O)-CH₃ (CPS-179, reference example);
R' is -CH₂-CH(OH)-; R" is -H; Y is phenyl; and substituents on Y are 3"-OH (CPS-192, reference example) or
R' is -CH₂-CH(OH)-; R" is -CH₃; Y is phenyl; and substituents on Y are 3"-OH (CPS-195, compound of the invention).

Some preferred examples of compounds of Formula 1 are:
2-Isopropyl-5-methyl-cyclohexanecarboxylic acid [2'-hydroxy-2'-(3"-hydroxyphenyl)-ethyl]-*N*-methyl-amide (compound of the invention); and
2-Isopropyl-5-methyl-cyclohexanecarboxylic acid [2'-(4"-acetyl-2"-hydroxymethylphenyl)-ethyl]-amide (reference example).

Some especially preferred examples of compounds of Formula 1 are:

| Code No. | Structure |
|---|---|
| CPS-195 (compound of the invention) | 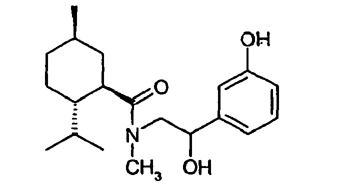 |
| CPS-179 (reference example) | 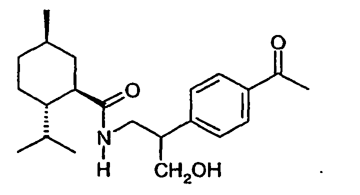 |

Also disclosed are compounds where the p-menthoyl moiety is conjugated to an α-adrenergic receptor vasoconstrictor agonist or to a phenylethanolamine or a phenylpropanolamine derivative that indirectly releases norepinehrine from nerve endings and exert a vasoconstrictor effect. These α-adrenergic vasoconstrictor substances include ephedrine, epinephrine, phenylephrine, methoxamine, metaraminol, α-methylnorepinephrine, norepinephrine, octopamine, phenylethanolamine, phenylpropanolamine, pseudoephedrine, and synephrine (see Table 1).

### Hypothesis on Refreshment and Relief of Sensory Discomfort and Novelty.

The inventor believes that the *N*-(substituted-arylhydroxyalkylidene)-cycloalkyl carboxamides), abbreviated here as "*N*-arylₛhydroxyalkylidene-cCarboxamide", and synonymous with "cycloalkyl carboxylic acid-aryl-R'-substituted amide", where R' is hydroxylalkyl (and the corresponding terminology is either the phenyl-alcohols or as phenylhydroxyethylamide, phenylhydroxypropylamide) act on ion channel receptors to stimulate discharge of peripheral sensory neurons. The class of ion channel receptors may belong to the TRP (transient receptor potential) family of proteins that mediate the effects of cooling on sensory neurons (TRP-M8, TRP-A1). But the precise identities of such receptors have not yet been established. When the nerve fibers, most likely Aδ cold fibers, relay signals to the spinal cord and brain, these signals generate sensations of coolness and refreshment. If pathological conditions are present, these signals also suppress perception of signals for tissue irritation, for pruritus, and for acute and chronic pain.

Without being limited by theory, an analogy of operation is as though there were three telephone lines in the tissues, each with a different dialing mechanism and cable conduction system. One is for touch and pressure that is fast conducting. One for coolness and cold that is somewhat slower (Aδ conducts at about 2 to 6 meters/sec). One is for irritation, itch and pain that conducts slowly (<2 meters/sec, primarily C-fibers). It is known that acupuncture and scratching may relieve pain and itch via vibration and pressure, respectively, and that this process takes place at the level of the spinal cord (the so called "gate-control theory of pain"). In the analogy, one of two telephone lines interferes with the signaling of the other, but at the central exchange. Continuing the analogy of this disclosure, the inventor proposes the use of compounds of this discovery as the dialing mechanism for stimulating the telephone line responsible for signals of coolness and cold. Using this new telephone line, the inventors anticipates refreshing and cool signals to be generated and, in the presence of inflammation and injury, an antinociceptive effect to be achieved that has sensory benefit.

The compounds of this discovery are active at µg to mg/mL (nano to microM) concentrations when applied to the topical surfaces of the body. By "topical", it is meant that the application is onto surfaces of the body in contact with air, which includes the skin, the eye surface, the upper and lower respiratory tract, and the entrance and exit of the gastrointestinal tract, that is, the oral cavity, and the anorectum.

A second feature of these compounds is a longer duration of action, on the order of several hours, relative to compounds such as (-)-menthol and WS-5, which are active for less than one hour. The longer duration of action was discovered by using a bioassay, specifically, the application of the test compounds to the skin of test subjects, and not by using taste thresholds for coolness or by using in vitro receptor assays.

### Tests for Activities of New Compounds

Pain was defined by Sir Charles Sherrington as "the psychic adjunct of an imperative protective reflex". Psychic events such as cooling, irritation, itch, and pain cannot be expressed by animals (animals cannot say "ouch" or that "it itches") so the sensory effects of chemicals must be indirectly inferred. Receptor assays,-based on cells transfected with the gene for TRP-M8 or TRP-A1, may be used as a model of sensory processes. These data, such as EC50 (median effective concentration for producing a half-maximal response), are precise. But, as noted below, these assays give no information on how long and for what quality of sensations is occurring on human subjects. The best information on the pharmacological properties of these chemicals must therefore be derived from human experiment.

In humans, Rowsell et al. tested the properties of *N*-arylₛ-cCarboxamide by putting filter paper (1 x 1 cm), impregnated with a known amount of compound onto the dorsal surface of the tongue of the test subject. After 30 seconds, the subject was required only to report presence or absence of a cooling effect. These data were reported as threshold values in µg of test substance.

The inventor has found that the cooling and sensory properties of a given compound, including the N-arylₛhydroxyalkylidene-cCarboxamide of this discovery, may be best obtained by suspending or dissolving a test substance in an ointment (usually Aquaphor® ointment which is 41% petrolatum, and the rest mineral oil, ceresin and lanolin alchol) and applying the emulsion (e.g., 0.2 to 0.5 cm³) onto the skin surface. A reliable place for topical application is on the upper lip, above the vermillion border of the lips, on the philtrum, lateral to the philtrum until the nasolabial folds, and on the lower nostrils. This part of the face is known to be densely innervated with cold receptors, second only to the surface of eyeball. Tingling, cool and cold sensations may be experienced and rated for intensity. This test is quite accurate for predicting the cooling activity of candidate N-arylₛhydroxyalkylidene-cCarboxamide drugs, but most importantly, it also measures the duration of drug action on the sensory receptors. The duration of action is not measured in a receptor assay and is difficult to quantify on the tongue because of the dynamic fluid conditions in the oral cavity and the presence of taste factors that affect thermosensation.

### Qualitative Aspects of Cool and Cold Intensity

The temperature of the skin and its environs gives rise to sensations that are qualitatively distinct. Thus, the normal skin temperature is 93°F (33.9°C) and when water is applied to the skin it is called "tepid" at 80°F to 93°F (26.7°C to 33.9°C); "cool" between 65°F and 80°F (18.3°C and 26.7°C), "cold" at 55°F to 65°F (12.8°C to 18.3°C), and "very cold" below 55°F (12.8°C) (see, e.g., Bierman, 1955. "Therapeutic Uses of Cold", J. Amer. Med. Assoc., Vol. 157, pp. 1189-1192). The lowering of skin temperature is accompanied by behavioral and emotional responses. Thus, at room temperatures at or below 65°F, an individual frequently seeks to turn on the thermostat. At ambient skin temperatures at or below 55°F, the sensations are aversive and accompanied by affect; that is, the person considers these cold sensations to be unpleasant, seeks to escape the environment, and may become angry; hostile, or-malaised if escape is not possibly. The emotional response is also influenced by the circumstances and the site of exposure. For example, ice cream inside the mouth is not aversive on a short-term basis, but an ice cube placed on the skin for more than few minutes is unpleasant. On the other hand, breathing cool air between 65°F and 80°F is refreshing and alerting, especially if there is inflammation in the upper respiratory tract or if the skin temperature is elevated above normal.

### Structure-Activity Relationships

The inventor has found the *N*-arylₛ-cCarboxamide, CPS-112 (also known as WS-12), with a 4-methoxy-substitution on the phenyl ring, is active at sub-micromolar concentrations on the TRP-M8 receptor assay. This compound elicits strong cold sensations when applied at 1 to 2% to the philtrum and its environs. The sensations are more cold than cool, and have the sting and harshness of high concentrations of menthol. The duration of action of a 2% preparation peaks at 30 minutes and is dissipated at the end of one hour. CPS-112 was therefore a disappointment in spite of its high potency on the receptor assay.

For topical uses, either as a cosmetic or as a therapeutic, it is desirable to have more coolness than cold, more cold than intense cold, and to have a duration of action that permits a practical application. For example, in cosmetic use, if the added compound is used to reduce the irritation produced by retinoids or α, or w- fatty acids, then the duration of action should be at least three hours. The sensation should be of coolness and not of cold. These qualitative aspects of sensation are met by CPS-116 and CPS-125, but not by CPS-112, which produced sensations of cold, but of brief duration. If the ointment is to have therapeutic value in the pruritus caused by xerosis in the elderly, in atopic eczema, or in perianal inflammation, then the duration of action is preferably more than six hours. Again, the philtrum method of testing gives an answer for the duration of action and the quality of sensation which is not obtainable with the tongue threshold or in vitro receptor methods.

### Method for Drug Design

To further improve duration of drug action in practicing this discovery, two design features were conceived and then implemented. One was to link the pharmacophore (defined as the functional unit on the molecule responsible for its drug action) to a vasoconstrictor element that will reduce diffusion of the coolant portion of the molecule away from its site of action. A second design feature was to use computer database software to predict distribution and safety of the molecule after structural modifications. A satisfying result of these ideas was the finding that the substitution of a hydroxyalkylidene group for the alkylidene group in the N-arylₛalkyl-cCarboxamide significantly improved the duration of action and reduced penetration into the brain.

For the first concept, in one set of the inventive embodiments, the inventor coupled the coolant portion of the molecule, residing in the p-menthane carbonyl group, to a phenylhydroxyalkylamine unit that has actions on blood vessels. Substituted phenylalkylamines and phenylhydroxyalkylamines are substances used for controlling blood vessel diameter and encompass molecules such as norepinephrine, epinephrine, ephedrine, isoproterenol, metaraminol, methoxamine, and phenylephrine. These compounds encompass α-adrenergic receptor agonists and substances that release norepinephrine from nerve endings and are collectively called sympathomimetic drugs because they mimic the actions of stimulation of the sympathetic nervous system. These compounds are listed, for example, in standard texts such as Goodman and Gilman, Pharmacological Basis of Therapeutics, 11th Edition, Brunson et al., editors, in Table 10-1, "Chemical structures and main clinical uses of important sympathomimetic drugs", pp. 240-241.

Table 1 illustrates some suitable α-adrenergic receptor agonists, which can then be coupled, or conjugated by N-acylation with a coolant, such as 2-isoproply-5-methyl cyclohexanecarboxylic acid, to produce cooling agents with very long durations of action.

| Table 1 Structures of some suitable sympathomimetic drugs that act as α-adrenergic receptor agonists | | | |
|---|---|---|---|
| 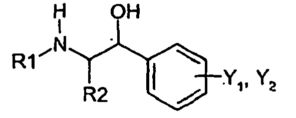 | | | |
| | | | |
| Chemical Name | R1 | R2 | Y₁ Y₂ |
| Epinephrine | CH₃ | H | 3',4'-dihydroxy |
| Norepinephrine | H | H | 3',4'-dihydroxy |
| Metaraminol | H | CH₃ | 3'-OH |
| phenylephrine | CH₃ | H | 3'-OH |
| Methoxamine | H | CH₃ | 2',5'-dimethoxy |
| α-Methylnorepinephrine | H | CH₃ | 3',4'-dihydroxy |
| Ephedrine | CH₃ | CH₃ | H, H |
| Octopamine | H | H | 4'-OH |
| Synephrine | CH₃ | H | 4'-OH |
| Pseudoephedrine* | CH₃ | CH₃ | H, H |
| Phenylpropanolamine* | H | CH₃ | H, H |
| Isoproterenol** | (CH₃)₂-CH- | H | 3',4'-dihydroxy |

| | | | |
|---|---|---|---|
| * = indirect acting sympathomimetic. ** = an α-and β- adrenergic receptor agonist. | | | |

The use of vasoconstriction to limit distribution and restrict the actions of local anesthetics on sensory fibers is known in pharmacological practice. Thus, to obtain nerve block.with a local anesthetic, such as used in dentistry, epinephrine is co-injected with procaine to limit the diffusion of the procaine into the bloodstream. Cocaine is sometimes preferred to procaine in nasal membrane surgery because cocaine also inhibits the neuronal re-uptake of norepinephrine, and hence cocaine has a dual action, local vasoconstriction and anesthesia, resulting is less blood loss during surgery. In the case of cocaine, vasocontrictive and local anesthetic properties are present in the same molecule. This concept of dual activity, however, has never been applied to design and produce a molecule with both coolant and vasoconstrictive properties.

Thus, in the case of CPS-195, where p-menthoyl chloride is conjugated to L-phenylephrine to form 2-isopropyl-5-methyl-cyclohexanecaboxylic acid (2'-hydroxy-2'-[3"-hydroxy-phenyl]-ethyl)-*N*-methyl-amide, the goal was to obtain a compound with prolonged cooling/antinociceptive action because (a) the parent is bioactive, and (b) the metabolites are also bioactive. The metabolite 2-isopropyl-5-methyl-cyclohexanecaboxylic acid (WS-1) is known to have mild cooling activity, and the second metabolite, L-phenylephrine, is a potent vasoconstrictor. The result was a compound with a surprisingly long duration of action - on the order of 4 hours after a single dose of 2 mg applied to the surface of the tongue or on to the philtrum.

An additional advantage of CPS-195, from a toxicological viewpoint, is that the pathways of phenylephrine (syn. m-synephrine) metabolism in humans are known. Thus, phenylephrine is converted to m-hydroxymandelic acid, m-hydroxyphenylglycol, phenylephrine sulfate, and phenylephrine glucuronide. This increases knowledge about the safety of the molecule because its potential metabolites have been characterized.

In the second concept, the importance of the hydroxyalkylidene group on access and residence of the agonist molecule at the target sites was analyzed. A key pharmacokinetic determinant of drug distribution and delivery on the skin is the octanol/water partition coefficient. Thus, the logarithm of the octanol/water partition coefficient (log P) in the range of 2.0 to 4.0 is considered ideal for activation of cool receptors in the skin and mucous membranes. For a systemic antinociceptive agent, the range is more restrictive, in the range of 2.5 to 3.5, because it is not desirable for the drug to cross the blood-brain-barrier (BBB). Sophisticated ADMET analysis programs, such as are available from Simulation-Plus (Lancaster, California) can predict drug passage across the BBB, based on the parameters described by Crivori et al., 2000, "Predicting blood-brain barrier penetration from molecular structure", J. Med. Chem., Vol. 43, pp. 2204-2216. In the examples, compounds were selected based on a low likelihood of passage across the BBB.

An analysis of the structure-activity relationships from the present studies indicates that the presence of a hydroxyalkylidene group, preferably hydroxymethylidene or hydroxyethylidene, on N-substituted-p-menthane-carboxamides confers the desirable quality of low penetration across the BBB yet retaining cooling activity. The presence of polar entities on the molecule in the addition to the hydroxyalkylidene group, e.g., hydroxyl or carboxyl or sulfonamido, also contributes to desirable ADME (absorption, distribution, metabolism, excretion) properties that will permit use as a systemic agent and yet reduce penetration of the BBB. This analysis of ADME properties is a preferred mode of practicing aspects of this discovery and these ADME properties were determined by programs designed to simulate and model pharmacokinetic parameters of ideal drug candidates.

### Delivery and Therapeutic Uses of N-Arylsₛhydroxyalkylidene-cCarboxamides

In practicing this discovery, the *N*-arylₛhydroxyalkylidene-cCarboxamide compounds are incorporated into topically suitable formulations, applied topically to inflamed skin and mucous membranes, and will typically relieve itch, irritation and pain. By "topical" it is meant application onto surfaces of the body in contact with air, which includes the skin, the eye surface, the upper and lower respiratory tract, and the entrance and exit of the gastrointestinal tract, that is, the oral cavity and the anorectum. Suitable topical formulations, for example, include compositions such as powders, pastes, lotions, liniments, creams and ointments, and cosmetic preparations.

In formulating topical compositions to practice this discovery, the *N*-arylₛhydroxyalkylidene-cCarboxamide compound may be incorporated into a vehicle that by itself may be inert or may contain other active ingredients (e.g., a flavor or a glucocorticosteroid). A wide variety of vehicles will be suitable, depending upon the particular product involved, such vehicles including solids, liquids, propellants, emulsions, foams and gels. Typical vehicles include oils and fats such as hydrocarbon oils, fatty acid esters, long chain alcohols and silicone oils; finely divided solids such as starch or talc; low-boiling hydrocarbons; gums and natural or synthetic resins. For applications to the ocular surface or to the upper or respiratory tract, the compound may be packaged in unit dose dispensers. For the lower respiratory tract a pressure activated metered dosage inhaler or nebulizer may be used.

Therapeutic indications for which a topical formulation may be beneficial include irritation, itch and pain from various forms of dermatitis (atopic, contact and irritant); pain from burned, traumatized or irritated skin, from procedures related to wound debridement; itch and discomfort from skin infections, insect bites, sunburn, actinic keratoses, basal cell carcinoma, pruritus due to xerosis; cheilitis or itching of the lips from cold sores; pruritus ani, hemorrhoidal discomfort, pain from anal fissures, pain or itch from anal fistulas, pain from hemorrhoidectomy, perineal inflammation, anogenital skin inflammation and discomfort due to various local causes such as incontinence, diaper rashes, perineal inflammation; vulval pruritus and pain (e.g., from candidiasis or idiopathic, such as vulva vestibulitis and vulvodynia), dyspaurenia, anogenital infections, including warts and sexually transmitted diseases, viral infections of the skin (especially in immunocompromised patients); nostril and nasal or upper airway discomfort from breathing obstruction, e.g., rhinitis, asthma, bronchitis, emphysema and chronic obstructive pulmonary diseases, sleep apnea and snoring; conjunctivitis, pain from corneal abrasions, and pain from eye surgery.

The use of the inventive embodiments in an asthma medication is of special interest because a primary pathogenic process in asthma, hyper-responsiveness of airways, may be susceptible to an agent that affects sensory processes. Thus, an agent such as CPS-195 may be incorporated into an inhaled medication and delivered via a pressurized-metering dose inhaler (a standard form of delivery for asthma medications). The inventive embodiment may be the sole active ingredient or incorporated together with an anti-inflammatory corticosteroid, a bronchial smooth muscle relaxant and/or an antimuscarinic agent.

If the target is to be reached via the bloodstream, an oral formulation is designed to be optimally absorbed from the gastrointestinal tract and to achieve steady blood or plasma levels. Here again, a simple gelatin capsule or an enteric coated pill or capsule, designed for optimum dissolution at a given pH, is a familiar formulation to practitioners skilled in the art. Extensively used chemicals for enteric coating are cellulose acetate phthalate, methacrylic acid ester copolymers with acidic ionizable groups, and polyvinyl acetate phthalate. Standard coating ingredients are widely sold under the trademark of Eudragit® (Degussa Chemicals, Inc.). Dosage forms coated with methacrylic acid polymers dissolve in the ileum at about pH 6.8, and in the terminal ileum and caecum at about pH 7.2. In general, coating thicknesses of about 25 to 200 µm, and especially 75 to 150 µm, are preferred using about 3 to 25 mg, preferably 8 to 15 mg of acidic coating material per square centimeter of tablet or capsule surface. The precise coating thickness will however depend upon the solubility characteristics of the material used and the site to be treated.

Therapeutic indications for which a systemic formulation may be beneficial include irritation, itch and pain from various forms of pathology. For example, systemic pruritus occurs frequently in patients undergoing renal dialysis and in patients with certain forms of liver failure. It is believed that certain endogenous substances (e.g., enkephalin peptides, bile acids) are not adequately cleared or released during kidney or liver dysfunction and these substances then act on nerve endings to produce generalized itching. Current pharmacotherapy with opioid antagonists such naltrexone, peripheral kappa-opioid receptor agonists such as nalfurafine, substance P antagonists such as odsanteron, cannabinoid receptor agonists, antihistamines and other chemicals are of limited effectiveness in treatment. The rationale in this application is to use compounds with cooling properties, to deliver these agents into the bloodstream, and to target receptors on sensory nerves and epithelium, in order to reduce noxious sensory perception.

### Additional Applications

The *N*-arylsₛhydroxyalkylidene-cCarboxamide compositions described here have the desirable properties of non-irritancy, safety and long duration of action. Uses of a formulation containing the inventive embodiments would include conditions such as heat exhaustion and fatigue, nasal and eye irritation, obstructed breathing disorders, lower urinary tract disorders, heartburn, irritable bowel disease or the irritable bowel syndrome, generalized pruritus, and systemic pain. Itch and pain are common sensory discomforts associated with various disorders, a list wherein said active agent is intended for the treatment of a disorder would include and be selected from the group consisting of contact dermatitis, atopic dermatitis, seborrheic dermatitis, chronic dermatitis of the hands and feet, exfoliative dermatitis, stasis dermatitis; lichen simplex chronicus; diaper rash; bacterial infections including erysipelas, cutaneous abscesses, necrotizing subcutaneous infections, folliculitis, furuncles, hidradenitis suppurativa, carbuncles, paronychial infections, erythrasma; fungal infections including dermatophyte infections, yeast infections; parasitic infections; viral infections, disorders of hair follicles and sebaceous glands, acne, rosacea, perioral dermatitis; scaling papular diseases, psoriasis, pityriasis rosea, lichen planus, pityriasis rubra pilaris, benign tumors, moles, seborrheic keratoses, keloid; malignant tumors, basal cell carcinoma, squamous cell carcinoma, malignant melanoma, Kaposi's sarcoma; reactions to sunlight, sunburn, photosensitivity; pemphigus, vitiligo, albinism, drug-induced hyperpigmentation, ichthyosis, corns, actinic keratosis; pressure sores, erythema multiforme, erythema nodosum. Included are non-dermatological disorders, which respond to topical/transdermal/oral delivery of an active agent, such as localized pain, generalized pain, nociceptive pain, neuropathic pain, joint pain, muscle pain, back pain, cardiac pain, rheumatic pain, arthritis, ostheoarthritis, acute soft tissue injuries and sports injuries, pelvic pain, premenstrual syndrome (PMS), dysmenorrhea, endometriosis, and vulvodynia, candidal vaginitis, herpes simplex, genital ulcers and warts, dyspareunia and vaginismus, anal abscess/fistula, anal warts, inflammatory bowel diseases, Crohn's disease, hemorrhoids, perianal thrush, anal fissures, sexually-transmitted disease and non-sexually-transmitted vaginal and genital infectious disease.

### EXAMPLES

The following examples are provided solely to illustrate the present invention and are not intended to limit the scope of the invention, as described herein.

### Example 1

N-Acylated carboxamides can be synthesized by reacting a carboxylic acid with thionyl chloride and then conjugating the acid chloride to the appropriate amine. This is a general chemical procedure familiar to the practitioners of the art and can be accomplished without undue experimentation. In preparing a conjugate of this discovery shown by Formula 1, the carboxylic acid chosen, for example, may be (1*R*,2*S*,5*R*)-2-isopropyl-5-methyl-cyclohexanecarboxylic acid, the preferred enantiomer. This chemical is also known as WS-1 and may be synthesized from 1-menthol or purchased from commercial firms. WS-1 may be reacted with thionyl chloride to form the reagent p-menthoyl chloride, or it can be reacted directly in situ with the desired amine conjugate (that is, the vasoconstrictor). The resulting conjugated product shown by Formula 1, usually a white crystalline solid, may then be purified by column chromatography and isolated by solvent evaporation. These are standard procedures of the organic chemistry laboratory.

Many substituted arylhydroxyalkylamines may be obtained from commercial sources such Sigma-Aldrich Corp., St. Louis, MO. For example, phenylephrine HCl, metanephrine HCl, isoproterenol HCl, norepinephrine HCl, and octopamine HCl are listed in the 2005-2006 Aldrich Catalog. The acid chloride is reacted with the appropriate arylhydroxyalkylamine to form the desired N-arylsₛhydroxyalkylidene-cCarboxamide.

### Example 2

### Synthesis of 2-isopropyl-5-methyl-cyclohexanecaboxylic acid [2'-hydroxy-2'-(3'-hydroxyphenyl)-ethyl]-N-methyl-amide (compound of the invention)

L-Phenylephrine HCl ((*R*)-(-)-3-(1-hydroxy-2-methylamino-ethyl)-phenol. hydrochloride) was purchased from Aldrich Chemicals, Co., Milwaukee, Wisonsin. 1.0 g was dissolved in 28 mL diethyether and 1 mL double-distilled water and cooled to 0°C. A pinch of the catalyst diaminopyrimidine was added. 1.90 mL of p-menthoyl chloride was then added dropwise, followed by 2 mL of triethylamine. Clumps of white precipitate appeared in the mixture, which was stirred overnight at room temperature. The precipitate was dissolved with ethyl acetate, washed with double-distilled water, and dried over sodium sulfate. The organic phase was then evaporated under reduced pressure to yield the final product (1.8 g), which crystallized at room temperature. The expected molecular mass was then confirmed by mass spectroscopy and the absorption spectrum by nuclear magnetic resonance. This compound was given the code of CPS-195.

### Bioassay Procedures

For bioassay of CPS-195 on the skin, CPS-195 was stirred and dissolved in warm liquid Aquaphor® ointment to yield ointment concentrations of 0.25, 0.5, 1, and 2% wt/vol. After cooling, 50 to 80 mg of the solid ointment was placed on the tip of a plastic stick and applied to the skin above the upper lip, on the philtrum, and lateral to the philtrum up to the nasolabial folds, of test subjects and the onset and duration of cooling sensations recorded. Similarly, the reference compounds CPS-179, CPS-190, and CPS-192 were formulated and tested, and the results are shown in Figure 1. Each of the compounds produce cooling on the skin of the upper lip with duration of action of more than 1 hour.

The intensity of the subjective skin sensation is rated as 0, 1, 2 or 3 with 0 as no change, 1 as slight coolness, cold, or tingling, 2 as clear cut signal of coolness, cold, or tingling, and 3 as robust cooling or cold. The interval for recording sensations is at 5 minute intervals, until two successive zeroes are obtained. The results are averaged values of 4 to 6 separate trials in the same individual. The data were plotted using SigmaPlot (Systat Software, Point Richmond CA) and a smoothing function with a negative exponential was used for analysis and statistical fit of the results.

The onset of drug action was taken as the time to reach 2 units of coolness intensity, and offset of drug action was the time when coolness intensity drops below 1.5, after previously surpassing 2 units. The duration of cooling action was defined as the offset time minus the onset time, which in this case averaged 2 minutes. The quality of the sensation was also noted, such as refreshing coolness, cold, or if the sensation was accompanied by irritation (stinging or burning). The quality of the sensation was not rated for intensity.

CPS-195 produces sensations of coolness and cold on the skin of the upper lip with duration of action of 1.2, 1.8, 2.4, and 5.2 hours for the four concentrations (0.25, 0.5, 1 and 2%) tested. Compared to the reference compounds CPS-179, CPS-190, and CPS-192, it was the most potent.

Placement of 2 mg of CPS-195 on the dorsal surface of the tongue resulted in a pleasant cooling sensation within the oral cavity that lasted for the surprisingly long period of over 4 hours. The onset of cooling was 3 to 5 minutes, but once the effect was established, the entire oral cavity and pharyngeal surfaces (oro-, laryngo- and naso- pharynx) felt cool. No significant tastes, such as bitterness or saltiness, were associated with CPS-195 and the cool/cold sensations were pleasant and not aversive.

CPS-195 was formulated as a 3% wt/wt tablet in Ludipress®, a rapidly disintegrating direct compression excipient made by BASF Corp. The tablet size was 60 mg so each unit contained 1.8 mg of CPS-195. In normal individuals, this tablet, when placed on the surface of the tongue, produced the effects as described in the previous paragraph for the powder. In an individual with throat irritation and cough, the CPS-195 tablet reduced throat irritation-and hard an anti-tussive effect that lasted for-24 hours. The prolonged action of CPS-195 tablet was unusual and indicated that it had multiple applications in therapy. For example, applied onto the mucous membranes of the oral cavity it can soothe the discomforts of pharyngitis/laryngitis, oral mucositis, oral ulcers, and idiopathic facial pain. In the pharynx and upper airways, CPS-195 may be used to reduce or to treat cough, asthma, and chronic obstructive pulmonary disease. On the skin, CPS-195 can be used to treat the discomfort of severe acne, eczema, diabetic ulcers, and the like.

The ADME parameters of CPS-195 were computed and the results showed that it is a suitable candidate to be a topical or systemic antinociceptive drug. No toxicity is expected to occur, as phenylephrine (part of the conjugate) is a known over-the-counter medication used as a vasoconstrictor for the eye surface and as a decongestant on the nasal membranes. The synthesis of CPS-195, depending on the starting substrate, can generate two enantiomers with respect to the asymmetric carbon in the alkylidene group (-R'-). The herein-described drugs are meant to encompass, where applicable, any and all enantiomers, mixtures of enantiomers including racemic mixtures, solvates, different physical forms (e.g., crystalline solids, amorphous solids), metabolites, and the like.

The optically pure α-adrenergic agonists and other sympathomimetics are known and described in the art; see, for example, Patil et al., 1975, "Molecular geometry of adrenergic drug activity", Pharmacological Reviews, Vol. 26, pp. 326-392 and US Patent 6,664,424 (Booth et al., December 16, 2003).

Other compounds contemplated for conjugation with p-menthoyl chloride include octopamine, methoxamine, metanephrine, pseudoephredrine, and such entities as t-butyl-4-aminobenzoate, (R)-(-)-t-butylamino-1-phenylethanol, and (S)-(+)-t-butylamino-1-phenylethanol, all of which are available from Aldrich Chemical Co.

### Example 3

A number of compounds were synthesized or simulated to ascertain the importance of adding a hydroxylalkylidene group to pharmacokinetic parameters. Several programs were used for simulation, including a log P program designed for the College of Chemistry at the University of California at Berkeley. The data in Tables 2 and 3 were based on the ADME analysis programs from Simulation-Plus (Lancaster, California). This program uses the parameters described in Crivori et al., 2000, "Predicting blood-brain barrier penetration from molecular structure", J. Med. Chem., Vol. 43, pp. 2204-2216 for estimating drug passage across the BBB.

### Example 4

The presence-of a phenyl group increases the log P, reduces water solubility, and most compounds readily cross the BBB (see Table 2). The presence of an acidic function, such as a sulfonic acid or a salicylic acid, will increase protein binding and reduce entry into the brain; however, such acidic functions lower biological activity. CPS-125, a sulfonamide, has the desired properties of good biological activity and absence of penetration of the BBB. The presence of a hydroxyalkyl function on the phenyl, together with another polar function such as either acetyl or hydroxyl will reduce passages across the BBB. On the other hand, the methoxy function is not sufficiently polar to prevent passage.

| Table 2 R-CO-NH-Y, where Y = phenyl | | | | | |
|---|---|---|---|---|---|
| Code | Substituents on Y | log P | Water solubility | BBB entry | % protein bound |
| CPS-152 | 4'-CH₂SO₃H | 2.71 | 0.500 | low | 93 |
| CPS-146 | 2'-OH, 4'-C(=O)-NH₂ | 3.06 | 0.068 | high | 49 |
| CPS-129 | 3'-OH, 4'-SO₂NH-pyrmidine | 3.64 | 0.022 | low | 89 |
| CPS-131 | 2'-CH₂OH, 4'-C(=O)-CH₃ | 3.10 | 0.050 | low | 64 |
| CPS-132 | 2'-OH, 4'-CH₂OH | 3.15 | 0.110 | low | 48 |
| CPS-133 | 3'-CH₂OH, 4'-O-CH₃ | 3.46 | 0.070 | high | 64 |
| CPS-134 | 4'-CH₂OH | 3.58 | 0.050 | high | 68 |
| CPS-141 | 3'-OH, 4'-C(=O)-CH₃ | 3.68 | 0.013 | high | 53 |
| CPS-147 | 4'-SO₃-CH₃ | 3.71 | 0.088 | high | 62 |
| CPS-140 | 4'-C(=O)-CH₃ | 3.85 | 0.010 | high | 68 |
| CPS-125 | 4'-SO₂NH-pyrimidine | 3.86 | 0.017 | low | 96 |
| CPS-149 | 2'-OH, 4'-C(=O)-OH | 4.31 | 0.052 | low | 88 |
| CPS-309 | 2'-O-CH₃, 4'-O-CH₃ | 4.34 | 0.006 | high | 50 |
| WS-12 | 4'-O-CH₃ | 4.39 | 0.008 | high | 68 |
| CPS-306 | 4'-O-CH₂-CH₃ | 4.85 | 0.004 | high | 70 |
| CPS-111 | 4'-C(=O)-OCH₂-CH₃ | 4.90 | 0.004 | high | 67 |
| CPS-310 | 4'-O-(CH₂)₂-CH₃ | 5.39 | 0.002 | high | 72 |
| CPS-308 | 4'-O-(CH₂)₃-CH₃ | 5.93 | 0.001 | high | 74 |

In the above table, BBB denotes blood-brain-barrier; water solubility-is quoted in units of mol/L; and log P = log octanol/water partition coefficient. These parameters were obtained using a commercial computer analysis program for ADMET prediction.

All the compounds of Table 2 are reference examples.

### Example 5

As shown in Table 3, the presence of a hydroxyalkylidene group on -R'- and one or more polar groups (hydroxyalkyl, hydroxyl or acetyl) on the aryl ring decreases the likelihood of passage across the BBB. As illustrated by Figure 1, CPS-179, CPS-190, CPS-192 and CPS-195 produced cooling on the skin of the upper lip with duration of action of more than 1 hour at concentrations of 0.5% ointment. The ADME parameters for these chemicals (Table 3), and the calculated log P value of less than 3.5 is favorable for topical penetration into tissue targets. Protein binding is not a significant factor affecting the distribution of these compounds. Thus, CPS-179, CPS-190, CPS-192 and CPS-195 were deemed suitable as candidates for development as antinociceptive agents.

All the compounds of Table 3 are reference examples, except CPS-195.

| Table 3 R-CO-N(R")-R'-Y (R is (1*R*,2*S*,5*R*)-2-isopropyl-5-methyl-cyclohexyl and Y is phenyl) | | | | | | | |
|---|---|---|---|---|---|---|---|
| Code | R' | R" | Substituents on Y | log P | Water solubility | Entry into BBB | % protein bound |
| CPS-177 | -CH₂-CH(OH)- | -H | 4"-CH₂OH | 2.71 | 0.265 | low | 68 |
| CPS-190* | -CH₂-CH(OH)- | -CH₃ | 3",4"-OH | 2.57 | 0.361 | low | 52 |
| CPS-178 | -CH₂-CH(CH₂OH)- | -H | 3"-CH₂OH, 4"-C(=O)-CH₃ | 2.83 | 0.256 | low | 67 |
| CPS-191* | -CH₂-CH(OH)- | -CH₃ | 3"-CH₂-CH₂OH, 4"-C(=O)-CH₃ | 2.83 | 0.165 | low | 44 |
| CPS-179 | -CH₂-CH(CH₂OH)- | -H | 4"-C(=O)CH₃ | 2.98 | 0.066 | low | 68 |
| CPS-192 | -CH₂-CH(OH)- | -H | 3"-OH | 2.98 | 0.228 | low | 33 |
| CPS-193* | -CH₂-CH(OH)- | -CH₃ | 3"-OCH₃, 4"-C(=O)-CH₃ | 3.05 | 0.074 | low | 35 |
| CPS-194* | -CH₂-CH(OH)- | -CH₃ | 3"-OH, 4"-OCH₃ | 3.06 | 0.161 | low | 50 |
| CPS-180 | -CH₂-CH₂-CH₂- | -H | 3"-CH₂OH, 5"-CH₂OH | 3.26 | 0.270 | low | 70 |
| CPS-195* | -CH₂-CH(OH)- | -CH₃ | 3"-OH | 3.29 | 0.317 | low | 32 |
| CPS-183 | -CH(CH₂OH)- | -H | none | 3.41 | 0.070 | high | 72 |
| CPS-185 | -CH₂-CH₂- | -H | 2"-CH₂OH, 5"-OH | 3.55 | 0.252 | low | 68 |
| CPS-186 | -CH₂-CH₂-CH₂- | -H | 3"-CH₂OH, 4"-C(=O)-CH₃ | 3.57 | 0.073 | low | 69 |
| CPS-196* | -CH₂-CH₂- | -CH₃ | 3",4"-OH | 3.76 | 0.051 | high | 48 |
| CPS-187 | -CH₂-CH₂-CH(OH)- | -H | none | 3.85 | 0.086 | high | 73 |
| CPS-197* | -CH₂-CH₂- | -CH₃ | 3"-CH₂-CH₂OH | 3.85 | 0.086 | high | 27 |

| | | | | | | | |
|---|---|---|---|---|---|---|---|
| * The asterisked compounds in the first column are *N*-methylated (i.e., R" = -CH₃). In the above table, BBB denotes blood-brain-barrier; water solubility is quoted in units of mol/L; and log P = log octanol/water partition coefficient. These parameters were obtained using a commercial computer analysis program for ADMET prediction. | | | | | | | |

In summary, the inventor has found that *N*-acylation of direct and indirect acting sympathomimetic drugs with a cooling pharmacophore such as p-menthane carbonyl increases duration of drug action. It was also found that introduction of a hydroxyalkylidene function (e.g., hydroxymethylidene or hydroxyethylidene) into a molecular pharmacophore of a cooling agent has the beneficial effect of reducing the likelihood of this molecule crossing the blood-brain-barrier. Other functional groups that enhance polarity are hydroxyl and acetyl, but not alkoxy. Thus, the incorporation of a hydroxyalkylidene group allows the drug to be administered in hydrophilic medium for topical applications and increases the versatility for clinical applications. Topical delivery to the lining of the upper airways is of special interest. The insights into the molecular parameters of pharmacokinetics are utilized for the selection of ideal antinociceptive drug candidates.

## Claims

1. A compound for use in a method of treating sensory discomfort, wherein said compound is 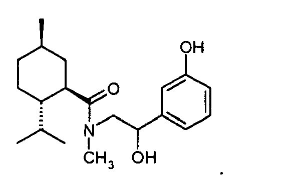 2-Isopropyl-5-methyl-cyclohexanecarboxylic acid [2'-hydroxy-2'-(3"-hydroxyphenyl)-ethyl-*N*-methyl-amide

2. A compound for use according to claim 1 wherein the sensory discomfort is itch.

3. Use of a compound in the manufacture of a medicament for the treatment of sensory discomfort, wherein said compound is: 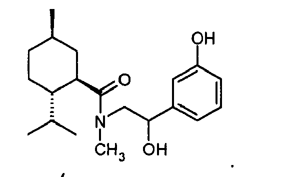 2-Isopropyl-5-methyl-cyclohexanecarboxylic acid [2'-hydroxy-2'-(3"-hydroxyphenyl)-ethyl]-*N*-methyl-amide

4. Use of a compound in the manufacture of a medicament according to claim 3, wherein the sensory discomfort is itch.

5. Use according to any one of claims 3 or 4 , wherein said medicament comprises said compound carried by a delivery vehicle.

6. Use according to claim 5, wherein said compound is present in an amount of from 0.1 wt. % to 2 wt. % of said delivery vehicle.

7. Use according to any one of claims3 or 4, wherein said medicament is a medicament for topical administration.

## Patentansprüche

1. Verbindung zur Verwendung in einem Verfahren zur Behandlung von sensorischen Beschwerden, worin die Verbindung 2-Isopropyl-5-methyl-cyclohexancarbonsäure[2'-hydroxy-2'-(3"-hydroxyphenyl)-ethyl]-N-methyl-amid ist:

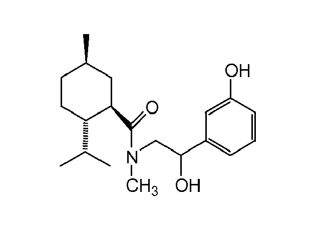

2. Verbindung zur Verwendung gemäß Anspruch 1, worin die sensorische Beschwerde Juckreiz ist.

3. Verwendung einer Verbindung für die Herstellung eines Medikamentes zur Behandlung von sensorischen Beschwerden, worin die Verbindung 2-Isopropyl-5-methyl-cyclohexancarbonsäure[2'-hydroxy-2'-(3"-hydroxyphenyl)-ethyl]-*N*-methyl-amid ist:

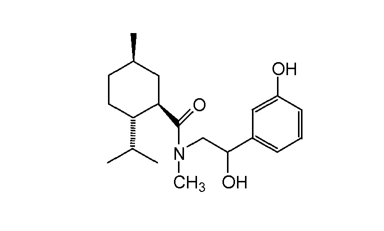

4. Verwendung einer Verbindung für die Herstellung eines Medikamentes gemäß Anspruch 3, worin die sensorische Beschwerde Juckreiz ist.

5. Verwendung nach einem der Ansprüche 3 oder 4, worin das Medikament die Verbindung umfasst, die durch einen Abgabeträger getragen ist.

6. Verwendung nach Anspruch 5, worin die Verbindung in einer Menge von 0.1 bis 2 Gew.-% des Abgabeträgers vorhanden ist.

7. Verwendung nach einem der Ansprüche 3 oder 4, worin das Medikament ein Medikament für die topische Verabreichung ist.

## Revendications

1. Composé à utiliser dans un procédé de traitement d'un inconfort sensoriel, dans lequel ledit composé est : 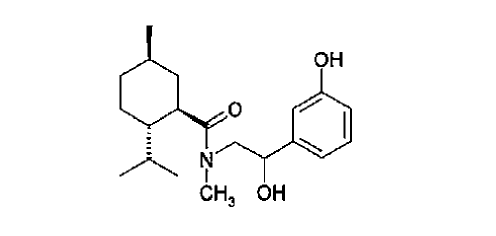 [2'-hydroxy-2'-(3"-hydroxy-phényl)-éthyl]-*N-*méthyl-amide d'acide 2-isopropyl-5-méthyl-cyclohexyanecarboxylique.

2. Composé à utiliser selon la revendication 1, dans lequel l'inconfort sensoriel est une démangeaison.

3. Utilisation d'un composé dans la fabrication d'un médicament destiné au traitement d'un inconfort sensoriel, dans laquelle ledit composé est : 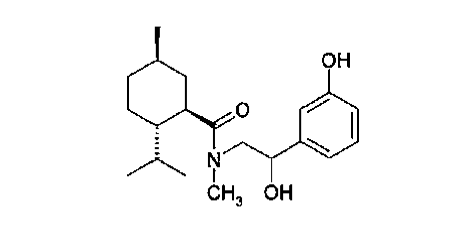 [2'-hydroxy-2'-(3"-hydroxy-phényl)-éthyl]-*N-*méthyl-amide d'acide 2-isopropyl-5-méthyl-cyclohexyanecarboxylique.

4. Utilisation d'un composé dans la fabrication d'un médicament selon la revendication 3, dans laquelle l'inconfort sensoriel est une démangeaison.

5. Utilisation selon l'une quelconque des revendications 3 et 4, dans laquelle ledit médicament comprend ledit composé porté par un véhicule d'administration.

6. Utilisation selon la revendication 5, dans laquelle ledit composé est présent dans une quantité de 0,1 % en poids à 2 % en poids dudit véhicule d'administration.

7. Utilisation selon l'une quelconque des revendications 3 et 4, dans laquelle ledit médicament est un médicament destiné à une administration topique.
